# EUROPÄISCHE PATENTSCHRIFT

(11) **EP 4 346 541 B1**
(45) Veröffentlichungstag und Bekanntmachung des Hinweises auf die Patenterteilung: **08.04.2026**
(21) Anmeldenummer: 22731556.1
(22) Anmeldetag: 31.05.2022
(51) Int. Cl.: A61B 1/00, A61M 25/01, A61B 34/30

(54) **BEWEGUNGSMECHANISMUS ZUM BEWEGEN EINES LÄNGLICHEN OBJEKTS**
MOVEMENT MECHANISM FOR MOVING AN ELONGATE OBJECT
MÉCANISME DE DÉPLACEMENT D'UN OBJET ALLONGÉ

(30) Priorität: 31.05.2021 DE 102021205547
(43) Veröffentlichungstag der Anmeldung: 10.04.2024
(73) Patentinhaber: Fraunhofer-Gesellschaft zur Förderung der angewandten Forschung e.V., 80686 München (DE)
(72) Erfinder: KARSTENSEN, Lennart, 68167 Mannheim (DE)
(74) Vertreter: Pfenning, Meinig & Partner mbB
(86) Internationale Anmeldenummer: PCT/EP2022/064700
(87) Internationale Veröffentlichungsnummer: WO 2022/253802

(56) Entgegenhaltungen:
- EP-A1- 2 517 666
- EP-B1- 2 626 033
- WO-A1-2012/071408
- WO-A1-98/48712
- DE-U- 1 868 409
- US-A1- 2014 276 389
- US-A1- 2015 090 057
- US-B1- 6 382 576

## Beschreibung

Die Erfindung betrifft einen Klemmaktor, der einen Hohlkörper mit einem Fluidvolumen aufweist, wobei der Hohlkörper eine u-förmige, rinnenförmige oder konkave Aufnahmebereichswand aufweist, die einen Aufnahmebereich umläuft und den Aufnahmebereich vom Fluidvolumen trennt. In den Aufnahmebereich ist ein längliches Objekt einlegbar. Die Erfindung betrifft außerdem einen Bewegungsmechanismus zum Bewegen zumindest eines länglichen Objekts mit einem solchen Klemmaktor.

In der Medizin müssen bei endovaskulären Eingriffen (z.B. zur Behandlung von Herzinfarkten und Schlaganfällen) schlauchartige und drahtartige Geräte (z.B. Katheter und Führungsdrähte) durch die Blutgefäße durch den Körper bewegt werden. Eine Telemanipulation oder eine automatische Manipulation dieser Katheter und Führungsdrähte bietet Vorteile, benötigt aber einen Manipulator. Dieser Manipulator sollte möglichst klein sein und idealerweise mehrere Geräte (z.B. Katheter/Führungsdrähte) konzentrisch bewegen können.

WO 2018/211184 A1 verwendet eine Einheit von zwei starren Backenpaaren, die ähnlich einem Greifzug/Mehrzweckzug das Gerät greifen und bewegen. Diese Lösung hat den Nachteil, dass große Aktoren (primär Elektromotoren) im Endeffektor benötigt werden, und eine Einheit zur Translation und Rotation eines Geräts sehr groß ist. Daher wird bisher nur der Führungsdraht mit Translation und Rotation bewegt und der Katheter als Rapid Exchange Katheter nur translatorisch bewegt.

EP 3380180 verwendet einen pneumatischen/hydraulischen Aktor, um mit einem Greifzugmechanismus die Translation einer Nadel zu ermöglichen. Eine Einheit besteht aus zwei ringförmigen Klemmaktoren und einem Balgaktor dazwischen. Die Klemmaktoren greifen abwechselnd die Nadel, und der Balgaktor sorgt währenddessen für die Translation. Hier besteht das System aus einer 3D-gedruckten Einheit. Eine Rotation ist durch Drehen der gesamten Einheit möglich und die Anwendung ist auf Nadeln beschränkt.

US 6,726,675 B1 verwendet als Aktor primär zwei Rollen, zwischen die das Gerät eingespannt wird. Translation wird durch ein Drehen der Rollen ermöglicht, Rotation durch ein Verkippen der Rollen. Diese Lösung hat den Nachteil, dass große Aktoren (primär Elektromotoren) im Endeffektor benötigt werden und eine Einheit zur Translation und Rotation eines Geräts sehr groß ist. Daher wird bisher nur der Führungsdraht mit Translation und Rotation bewegt und der Katheter als Rapid Exchange Katheter nur translatorisch bewegt.

NL 1043050 verwendet als Aktoren ein Set aus Rollen für die Translation, die über Zahnräder gedreht werden. Das Wirkprinzip ist ähnlich zu US 6,726,675 B1, der Aufbau der Rollen aber anders. Dieses Patent soll vermutlich Interventionen im MRT ermöglichen und mit dem MRT tauglichen, pneumatischen Schrittmotor von Soteria zusammen als Manipulator verwendet werden.

WO 2005/117596 verwendet ein ähnliches Prinzip wie NL 1043050, indem Translation des Katheters/Führungsdrahtes durch ein Rollenpaar ermöglicht wird und für die Rotation das gesamte Rollenpaar gedreht wird. Es werden ebenfalls Zahnräder für die Übertragung der Bewegung verwendet, der Aufbau ist aber anders.

DE 102004007935 verwendet zwei Sets von je zwei Rollenaktoren, die einen Führungsdraht/Katheter einspannen. Ein Set führt die Translation aus und ein zweites dahinter am Instrument angebrachtes Set unabhängig davon die Rotation. Unterschied zu US 6,726,675 B1 und NL 1043050 sind die zwei Sets an Aktoren anstelle des Drehens der Rollenaktoren für die Translation. Nachteil ist die Reibung, die zwangsläufig an den Aktoren auftreten muss.

US 2007/0156123 A1 spannt einen abwinkelbaren Katheter in eine Kassette ein, in der Seile auf Rollen eine Spitze aktuieren können. Translation wird durch Verfahren auf einer Linearschiene ermöglicht. Eine Rotation an der Basis des Katheters ist nicht nötig, weil die Spitze manipuliert werden kann. Abgesehen von der Manipulation von Kathetern keine Ähnlichkeit zu dieser Erfindungsmeldung.

WO 2017/220010 A1 spannt einen abwinkelbaren Katheter in eine Apparatur ein, um diesen zu manipulieren. Translation wird durchgeführt, indem die ge samte Apparatur auf einer Linearschiene bewegt wird. Eine Rotation des Katheters wird über pneumatisch/hydraulische Kolbenaktoren ermöglicht. Das Abwinkeln des Manipulators wird ebenfalls durch Kolbenaktoren ermöglicht, die den Abwinkelmechanismus des Katheters betätigen. In beiden Fällen wird eine dünne Schicht eines elastischen Materials unter Druck gesetzt, um sich auszudehnen und gleichzeitig dicht zu bleiben. Aber die erzielte Wirkung des unter Drucksetzens ist eine grundsätzlich andere.

EP 2 517 666 A1 offenbart eine Vorrichtung zum Halten eines medizinischen Instruments. Die Vorrichtung weist zwei Klemmelemente auf. Mindestens ein Klemmelement ist auf ein anderes Klemmelement bewegbar, sodass das medizinische Instrument zwischen den Klemmelementen geführt und/oder fixiert werden kann.

DE 18 68 409 U offenbart eine Klemme, die mit Rasterfixierung an ihrem Ende mit zwei schalenförmigen Zangen ausgestattet ist. In jede schalenförmige Zange ist ein U-förmiger Ballon eingesetzt, der einige pilzartige Ausstülpungen zu seiner Fixierung in den Schalen trägt und mit einem Anschlussschlauch zum Aufblasen versehen ist.

WO 98/48712 A1 offenbart eine kugelförmige Halterung mit einem Durchgang. In dem Durchgang kann ein medizinisches Instrument befestigt werden.

US 6 382 576 B1 offenbart eine Klemmvorrichtung. Die Klemmvorrichtung umfasst eine aufblasbare Blase, die sich beim Aufblasen gegen ein Trägerelement ausdehnt oder sich um dieses herum verengt, um ein Instrument an das Trägerelement zu klemmen.

EP 2 626 033 B1 offenbart eine Vorrichtung, die eine Steuerung zum Steuern einer Ablenkung einem distalen Ende eines Katheters umfasst.

US 2015/090057 A1 offenbart eine abnehmbare Katheterbetätigungsmanschette, die einen Katheterbetätiger am Griff eines Katheters mit einer Katheterpositionierungsvorrichtung verbinden kann.

WO 2012/071408 A1 offenbart ein System zum Betreiben eines Katheters mit einem distalen Ende, das dazu geeignet ist, im Körper navigiert zu werden, und einem proximalen Ende, das einen Griff mit einer verschiebbaren Steuerung und einer drehbaren Steuerung zum Einwirken auf das distale Ende aufweist.

US 2014/276389 A1 offenbart ein System zum Betreiben eines länglichen medizinischen Instruments.

Aufgabe der vorliegenden Erfindung ist es, einen Bewegungsmechanismus anzugeben, mit dem die Probleme des Standes der Technik behoben werden können.

Die Aufgabe wird gelöst durch den Bewegungsmechanismus nach Anspruch 1.

Die jeweiligen abhängigen Ansprüche geben vorteilhafte Weiterbildungen des erfindungsgemäßen Bewegungsmechanismus an.

Erfindungsgemäß wird ein Klemmaktor zum Halten eines länglichen Objektes angegeben, der einen Hohlkörper mit einem Fluidvolumen aufweist. Unter einem Fluidvolumen kann hierbei ein Volumen verstanden werden, in dem ein Fluid, also eine Flüssigkeit und/oder ein Gas, haltbar ist. Das Fluidvolumen wird durch den Hohlkörper gebildet, ist also ein Volumen in dem Hohlkörper, der durch das Fluidvolumen zum Hohlkörper wird. Der Hohlkörper hat eine als Aufnahmebereichswand bezeichnete Wand, die eine Form hat, die als u-förmig, rinnenförmig oder konkav bezeichnet werden kann. Im letzteren Fall wäre sie vom Aufnahmebereich aus gesehen konkav. Die Bezeichnungen der Form der Aufnahmebereichswand als u-förmig, rinnenförmig und konkav können als synonym angesehen werden.

Die Aufnahmebereichswand umläuft einen Aufnahmebereich, in den das längliche Objekt einlegbar ist. Die Aufnahmebereichswand kann als zwei Schenkel aufweisend angesehen werden, die in einem Verbindungsbereich miteinander verbunden sind. Da sich die Aufnahmebereichswand entlang des Aufnahmebereichs erstreckt, sind die Schenkel Flächen, die sich entlang des Aufnahmebereichs erstrecken. Diese Flächen können auch gekrümmt sein. In einem Schnitt durch die Aufnahmebereichswand in einer Ebene senkrecht zu einer Richtung der Erstreckung des Aufnahmebereichs kann sich die Form eines zweidimensionalen U ergeben, dessen Schenkel die Schnitte der Schenkel der Form der Aufnahmebereichswand sind.

Die beiden Schenkel sind an ihrem einen Ende in einem Verbindungsbereich miteinander verbunden und können sich dann von dort aus in Richtung vom Verbindungsbereich weg, hier auch als Richtung des Schenkels bezeichnet, erstrecken. Der Verbindungsbereich kann dabei selbst eine Erstreckung in der zur Erstreckungsrichtung des Aufnahmebereichs senkrechten Ebene haben. Es ist aber auch möglich, dass der Verbindungsbereich lediglich eine Linie oder Gerade ist, an dem die Schenkel aneinander stoßen. Eine solche Form kann auch als V-Form bezeichnet werden, die hier als Spezialfall der U-Form, Rinnenform bzw. konkaven Form angesehen werden soll. Der Verbindungsbereich kann, sofern er eine Erstreckung in der besagten Ebene hat, gerade oder auch gekrümmt verlaufen, in letzterem Falle besonders vorteilhaft entlang einer Kreislinie gekrümmt, an der die Richtungen der Schenkel besonders bevorzugt tangential liegen können.

Erfindungsgemäß befindet sich zwischen den Schenkeln der Aufnahmebereichswand ein länglicher Aufnahmebereich, in den ein längliches Objekt eingelegt werden kann. Vorzugsweise erstreckt sich der Aufnahmebereich entlang einer Geraden (er ist also gerade), die besonders bevorzugt nicht die Aufnahmebereichswand schneidet.

Die Aufnahmebereichswand umläuft den Aufnahmebereich zumindest teilweise. Der Aufnahmebereich wird von der Aufnahmebereichswand teilweise umlaufen, so dass die Aufnahmebereichswand den Aufnahmebereich auf einem Teil seines Umfangs begrenzt. Die zwei Schenkel der Aufnahmebereichswand sind auf der dem Verbindungsbereich abgewandten Seite des Aufnahmebereichs nicht miteinander verbunden. Auf diese Weise kann ein längliches Objekt aus dieser Richtung in den Aufnahmebereich eingelegt werden. Es ist aber möglich, dass sich die Schenkel auf der dem Verbindungsbereich abgewandten

Seite berühren ohne miteinander verbunden zu sein. Zum Einlegen eines Objektes können sie dann auseinandergedrückt werden. Auch diese Form soll hier als U-Form, Rinnenform bzw. konkave Form angesehen werden.

Es ist bevorzugt, wenn die Aufnahmebereichswand den Aufnahmebereich zumindest soweit umläuft, dass sie bezüglich des Aufnahmebereichs gegenüberliegende Bereiche hat, deren Flächen parallel zueinander liegen. Auf diese Weise kann verhindert werden, dass das Objekt auf dem Aufnahmebereich herausgedrückt wird.

Vorteilhaft kann die Aufnahmebereichswand zumindest auf einem Teilabschnitt ihres Verlaufs um den Aufnahmebereich einen kreisförmigen, ovalen oder rechteckigen Querschnitt haben. Der Querschnitt kann vorteilhaft einem Querschnitt des länglichen Objektes entsprechen, wodurch dieses besonders sicher gehalten werden kann. Ein ovaler Querschnitt ist besonders vorteilhaft, da er ein leichtes Einlegen des länglichen Objektes in den Aufnahmebereich erlaubt und gleichzeitig das Objekt gut in zur Einlegerichtung senkrechter Richtung hält.

Erfindungsgemäß wird die Aufnahmebereichswand zumindest bereichsweise durch eine flexible Membran gebildet. Vorteilhafterweise kann die Membran auch elastisch sein. Wird ein Fluid in das Fluidvolumen mit einem Überdruck gegenüber dem Umgebungsdruck eingeleitet, so expandiert die flexible Membran in Richtung des Aufnahmebereichs und verkleinert diesen oder übt eine Druckkraft auf ein im Aufnahmebereich angeordnetes Objekt aus. Auf diese Weise kann das Objekt im Aufnahmebereich gehalten werden.

Die Schenkel der Aufnahmebereichswand müssen nicht gerade sein. Vorteilhaft ist beispielsweise eine in Richtung der Schenkel gekrümmte Form der Schenkel. Die Krümmung kann dabei vorteilhaft aus Sicht des Aufnahmebereichs konkav sein. Besonders vorteilhaft ist jedoch auch ein Verlauf der Schenkel, so dass die Aufnahmebereichswand den Aufnahmebereich um mehr als 180° umläuft.

Optional kann die Aufnahmebereichswand auf ihrer dem Aufnahmebereich zugewandten Oberfläche Noppen, Stege und/oder Rillen aufweisen, durch die ein Halt des länglichen Objekts im Aufnahmebereich verbessert werden kann. Stege und/oder Rillen können entlang des Aufnahmebereichs verlaufen und/oder entlang eines Umfangs um dem Aufnahmebereich.

Der Hohlkörper kann in jenen Bereichen, die sich an die Aufnahmebereichswand anschließen, äußere Oberflächenbereiche aufweisen, die in einem Winkel auseinanderlaufen. Jene Oberflächenbereiche, die auseinanderlaufen, können vorteilhaft gerade sein. Es ergibt sich hierdurch eine Form der Oberfläche des Hohlkörpers die dem Aufnahmebereich zugewandt ist, die sich zunächst nach außen hin öffnet, so dass ein von außen eingelegtes längliches Objekt zunächst durch eine sich verengende Trichterform geführt wird, bis es bei weiterer Verschiebung in Richtung des Verbindungsbereichs in den von der Aufnahmebereichswand z.B. kreisförmig umlaufenen Bereich kommt und dort gehalten werden kann.

Erfindungsgemäß weist der Klemmaktor außerdem einen Fluidanschluss auf, über den Fluid in das Fluidvolumen einleitbar und aus dem Fluidvolumen ableitbar ist.

In einer vorteilhaften Ausgestaltung der Erfindung kann die flexible Membran alle Seiten des besagten Fluidvolumens begrenzen. In diesem Fall kann also der Hohlkörper aus der flexiblen Membran hergestellt sein. Dieser Hohlkörper kann dann zusätzlich zu der flexiblen Membran auch weitere Elemente aufweisen, wie beispielsweise den Fluidanschluss, die in diesem Fall auch aus anderen Materialien als der Membran hergestellt sein können.

In einer vorteilhaften Ausgestaltung der Erfindung kann der das Fluidvolumen enthaltende Hohlkörper auch die Form eines Körpers haben, der eine äußere Oberfläche hat, deren Form von der Form des Fluidvolumens unabhängig ist. Zum Beispiel kann der Hohlkörper eine äußere Oberfläche haben, die die Form einer Zylinderfläche hat. Es sei angemerkt, dass der Begriff zylinderförmig hier im Sinne eines allgemeinen Zylinders verstanden werden soll, d.h. als eine

Form, die sich durch verschieben einer ebenen Kurve entlang einer Strecke ergibt. Die ebene Kurve kann dann zum Beispiel Kreisbogenform haben, mit einem die Enden des Kreisbogens verbindenden geraden Abschnitt. Vorzugsweise erstreckt sich der Kreisbogen dabei über einen Winkel von größer als 180° um den Aufnahmebereich. Alternativ kann die ebene Kurve auch ein Rechteck sein. Die äußere Oberfläche kann, vorteilhaft in einem ebenen Bereich, einen zur Längsachse des Aufnahmebereichs parallelen Einschnitt aufweisen. Dabei können die Wände des Einschnitts in die Aufnahmebereichswand übergehen, die das Fluidvolumen begrenzt. Vorteilhafterweise kann sich hierbei im Bereich des Einschnitts die oben beschriebene Trichterform ergeben. Durch diesen Einschnitt kann dann das längliche Objekt in den Aufnahmebereich eingeführt werden. Jener Oberflächenbereich des Hohlkörpers, der nicht den Einschnitt aufweist, der also beispielsweise einen kreisbogenförmigen Querschnitt hat, kann auch andere Formen aufweisen. Diese Form kann zum Beispiel der Form einer Innenwand eines Verbindungselements entsprechen, in das der Klemmaktor einsetzbar ist.

Die Form des Fluidvolumens kann in jenen Bereichen, die nicht durch die Aufnahmebereichswand begrenzt werden, frei gestaltet werden, zum Beispiel mit besagter Zylinderflächenform oder auch mit rechteckigem Querschnitt. Das Fluidvolumen kann also eine U-Form haben, deren dem Aufnahmebereich abgewandten Außenwände eine beliebige Form haben und deren dem Aufnahmebereich zugewandten Innenwände den Aufnahmebereich umlaufen, zum Beispiel entlang eines Kreisbogens.

Vorzugsweise erstreckt sich der Aufnahmebereich entlang einer Geraden. Die Richtung dieser Geraden ist die Längsrichtung des Aufnahmebereichs. Diese Gerade wird dann normalerweise senkrecht auf der besagten Ebene liegen, in der sich die Richtungen der Schenkel der U-Form, Rinnenform bzw. konkaven Form erstrecken. Ein längliches Objekt, das in den Aufnahmebereich eingelegt wird, kann dann mit seiner Längsrichtung koaxial zu der Geraden liegen, entlang der sich der Aufnahmebereich erstreckt. Dass sich der Aufnahmebereich entlang einer Geraden erstreckt, kann insbesondere bedeuten, dass der Aufnahmebereich entlang der Geraden durchgängig ist, was insbesondere bedeutet, dass diese Gerade nicht die Aufnahmebereichswand und das Fluidvolumen schneidet.

In einer optionalen Ausgestaltung kann das Fluidvolumen durch eine Wand begrenzt werden, die in einem Zustand, in dem keine äußeren Kräfte auf sie wirken, also insbesondere keine Kraft durch ein Fluid im Fluidvolumen, eine Form hat, die durch Verschiebung einer geschlossenen ebenen Kurve entlang eines um eine Mittelachse gekrümmten Verschiebewegs entsteht. Die Mittelachse kann dabei gerade koaxial sein zu der beschriebenen Geraden, entlang der der Aufnahmebereich verläuft. Dabei liegt die Mittelachse an jeder Position der Verschiebung außerhalb der von der Kurve umschlossenen Fläche sowie außerdem in der Ebene der Kurve. Der Verschiebungsweg umläuft in dieser Darstellung die Mittelachse über einem Winkelbereich von weniger als 360° und/oder die Enden des Verschiebungswegs schließen einen Winkel größer als Null um die Mittelachse ein. Der Winkel, den die Enden des Verschiebungswegs einschließen, ist dabei jener Winkel, der entsteht, wenn man die Enden des Verschiebungswegs mit dem Mittelpunkt des Verschiebungswegs verbindet. Diese Ausgestaltung ist insbesondere vorteilhaft, wenn die flexible Membran alle Seiten des Fluidvolumens begrenzt.

Vorzugsweise umläuft der Verschiebungsweg die Mittelachse um mehr als 180°, besonders bevorzugt um mehr als 270°. Wählt man als Verschiebungsweg einen Weg, der durch die innersten Punkte der geschlossenen Kurve gebildet wird, so kann dieser Weg den Aufnahmebereich auch um 360° umschließen, vorausgesetzt jedoch, dass dort, wo die entstehende Form der beiden Schenkel sich berührt, zwischen den Schenkeln keine stoffschlüssige Verbindung hergestellt wird. Auf diese Weise kann durch Auseinanderdrücken ein in den Aufnahmebereich einzulegendes Objekt zwischen den Schenkeln der entstehenden Form hindurch bewegt werden.

Vorteilhafterweise kann die Aufnahmebereichswand den Aufnahmebereich über einen Winkel von mehr als 180° umlaufen. Dadurch, dass das Fluidvolumen den Aufnahmebereich über einen Winkel von mehr als 180° umläuft, kann ein längliches Objekt, das im Aufnahmebereich gehalten wird, gegen Herausfallen gesichert werden.

Vorteilhafterweise kann die Oberfläche des Hohlkörpers Abschnitte aufweisen, die einen spitzen Winkel um den Aufnahmebereich einschließen. Auf diese Weise können diese Bereiche der Oberfläche zunächst auf den Aufnahmebereich hinzuverlaufen, dann den Aufnahmebereich im Bereich der Aufnahmebereichswand entlang eines Bogens, z.B. eines Kreisbogens, umlaufen und dann auf der gegenüberliegenden Seite sich wieder vom Aufnahmebereich entfernen. Es können dabei die Bereiche der Oberfläche, die auf den Aufnahmebereich hinzuverlaufen und vom Aufnahmebereich weg verlaufen, den genannten spitzen Winkel einschließen.

In einer optionalen Ausgestaltung der Erfindung können dem Aufnahmebereich zugewandte Wände des Fluidvolumens bzw. des das Fluidvolumen enthaltenden Hohlkörpers, also die Aufnahmebereichswand, parallel verlaufen zu den dem Aufnahmebereich abgewandten Wänden des Fluidvolumens oder des Hohlkörpers. Das Fluidvolumen kann also durch parallele Wände begrenzt werden bzw. es kann das Innere eines Hohlkörpers mit parallelen Wänden sein. Die parallelen Wände können hierbei ein u-förmiges Volumen als Fluidvolumen beschreiben.

In einer vorteilhaften Ausgestaltung kann der Klemmaktor zumindest ein Zentrierungselement aufweisen, das auf zumindest einer Seite des Aufnahmebereichs in Richtung dessen Längsrichtung angeordnet ist, wobei mit dem zumindest einen Zentrierungselement das längliche Objekt auf eine Mittelachse des Aufnahmebereichs zentrierbar ist, wobei vorzugsweise das zumindest eine Zentrierungselement eine Rinne oder eine Durchgangsbohrung aufweist, die koaxial zu der Mittelachse des Aufnahmebereichs verläuft. Besonders vorteilhaft kann ein solches Zentrierungselement auch auf beiden Seiten des Aufnahmebereichs in Richtung der Längsrichtung des Aufnahmebereichs angeordnet sein. Optional kann das zumindest eine oder beide der Zentrierungselemente auch als Teil des im folgenden beschriebenen Bewegungsmechanismus realisiert sein. Durch das zumindest eine Zentrierungselement wird erreicht, dass sich das längliche Objekt möglichst genau auf der Mittelachse des Aufnahmebereichs befindet. Dies ist insbesondere vorteilhaft, wenn das längliche Objekt durch den Aktor gedreht wird, da so sichergestellt wird, dass sich das längliche Objekt auf der Drehachse befindet, die vorteilhaft mit der Mittelachse zusammenfällt.

Sowohl jenes am Klemmaktor angeordnete Zentrierungselement als auch jenes am Bewegungsmechanismus angeordnete kann vorteilhaft zwei Teile aufweisen, die zwischen sich die besagte Durchgangsbohrung einschließen. Die beiden Teile können dann jeweils eine Rinne aufweisen und nach Einlegen des länglichen Objekts zusammengesetzt werden so dass die beiden Rinnen der Teile die Durchgangsbohrung bilden. Auch möglich ist eine Ausgestaltung, in der eines der Teile ein kovexes Element aufweist und das andere ein konkaves Element. Dies kann zum Beispiel vorteilhaft sein für unterschiedliche Durchmesser der länglichen Objekte, vorteilhaft in Verbindung mit einem leicht elastischen Schnappverschluss oder Ratschenverschluss.

Vorteilhaft können die beiden Teile so ausgestaltet sein, dass sie durch Magnetkraft, einen Schnappverschluss oder Ratschenverschluss zusammen halten. Vorzugsweise sind die zwei Teile auf einer Seite mit einem Scharnier verbunden und weisen auf der anderen Seite einen Verbindungsmechanismus auf.

Erfindungsgemäß wird außerdem ein Bewegungsmechanismus zum Bewegen zumindest eines länglichen Objekts angegeben, der zumindest einen Klemmaktor wie vorstehend beschrieben aufweist. Der Bewegungsmechanismus weist außerdem zumindest einen Bewegungsaktor auf, mit dem der Klemmaktor in Richtung einer Längsrichtung des Aufnahmebereichs verschiebbar ist und/oder um den Aufnahmebereich drehbar ist. Mit einem solchen Bewegungsmechanismus kann daher das längliche Objekt in Richtung seiner Längsrichtung verschoben werden und/oder um seine Längsrichtung gedreht werden.

In einer vorteilhaften Ausgestaltung kann der Klemmaktor in den Bewegungsaktor einsteckbar sein. Hierzu kann der Klemmaktor auf seiner bei bestimmungsgemäßer Anordnung dem Bewegungsaktor zugewandten Seite Verbindungselemente aufweisen, die mit Verbindungselementen am Bewegungsaktor in Eingriff bringbar sind.

In einer vorteilhaften Ausgestaltung kann der Bewegungsmechanismus zumindest zwei der Klemmaktoren aufweisen, deren Aufnahmebereiche vorteilhafterweise koaxial zueinander liegen. Auf diese Weise kann das längliche Objekt, vorteilhaft mit gerader Erstreckung, in beide Klemmaktoren eingelegt werden. In dieser Ausgestaltung kann der Bewegungsmechanismus für jeden der Klemmaktoren jeweils zumindest einen Bewegungsaktor aufweisen, mit dem der entsprechende Klemmaktor in Richtung der Längsrichtung des Aufnahmebereichs verschiebbar ist und/oder um den Aufnahmebereich drehbar ist.

In einer vorteilhaften Ausgestaltung kann der Bewegungsmechanismus an einem oder mehreren oder allen der Klemmaktoren jeweils zumindest ein Zentrierungselement aufweisen, das auf zumindest einer Seite des Aufnahmebereichs des entsprechenden Klemmaktors in Richtung dessen Längsrichtung angeordnet ist, wobei mit dem zumindest einen Zentrierungselement das längliche Objekt auf eine Mittelachse des Aufnahmebereichs zentrierbar ist, wobei vorzugsweise das zumindest eine Zentrierungselement eine Rinne oder eine Durchgangsbohrung aufweist, die koaxial zu der Mittelachse des Aufnahmebereichs verläuft. Besonders vorteilhaft kann ein solches Zentrierungselement auch auf beiden Seiten des Aufnahmebereichs in Richtung der Längsrichtung des Aufnahmebereichs angeordnet sein.

Ein Verfahren zum Bewegen eines länglichen Objektes wird angegeben, wobei das längliche Objekt in zumindest einen Klemmaktor eines Bewegungsmechanismus wie vorstehend beschrieben eingelegt wird. Es wird anschließend das Fluid in das Fluidvolumen des zumindest einen Klemmaktors eingeleitet und dann jener Bewegungsaktor aktuiert, der diesen Klemmaktor bewegt. Durch Einleiten des Fluids wird das längliche Objekt im Klemmaktor gehalten, so dass sich die Bewegung des Klemmaktors auf das längliche Objekt überträgt.

Eine besonders vorteilhafte Führung des Verfahrens erlaubt es, ein längliches Objekt kontinuierlich zu bewegen. Es weist hierfür der Bewegungsmechanismus einen ersten und einen zweiten der Klemmaktoren auf, die jeweils an einem Bewegungsaktor angeordnet sind. Es ist also der erste Klemmaktor an einem ersten Bewegungsaktor und der zweite Klemmaktor an einem zweiten Bewegungsaktor angeordnet. Dabei sind die Klemmaktoren mit den Bewegungsaktoren jeweils in Richtung der Längsrichtung ihrer Aufnahmebereiche verschiebbar. Es wird dann zunächst das längliche Objekt in die Aufnahmebereiche beider Klemmaktoren angelegt und dann die Klemmaktoren und die Bewegungsaktoren in einem Aktuierungsmuster aktuiert. Es werden dabei zumindest einmal wiederholt die folgenden Schritte in der Reihenfolge ihrer Nummerierung ausgeführt, wobei aufeinanderfolgende Schritte sich auch leicht überlappen können:
1) Der erste Klemmaktor wird in einer ersten Ausgangsposition aktuiert, so dass er das längliche Objekt greift. Die Ausgangsposition kann dabei eine Extremposition seines Bewegungsbereichs sein. Zum Beispiel kann der erste Klemmaktor sich ganz links befinden, wobei in der folgenden Beschreibung selbstverständlich links und rechts vertauscht werden können und von der Richtung der Betrachtung abhängen.
2) Der erste Bewegungsaktor bewegt den ersten Klemmaktor in eine Transportrichtung, die auf den zweiten Klemmaktor hin gerichtet ist. Zum Beispiel kann der erste Bewegungsaktor den ersten Klemmaktor nach rechts bewegen. Der erste Bewegungsaktor bewegt dabei vorzugsweise den ersten Klemmaktor bis zum maximalen Abstand von der ersten Ausgangsposition, was gleichzeitig die dem zweiten Klemmaktor nächste Position des Verschiebungswegs des ersten Klemmaktors sein kann.
3) Der erste Klemmaktor beendet dann seine Aktuierung, so dass er das längliche Objekt freigibt. Der zweite Klemmaktor wird in einer zweiten Ausgangsposition aktuiert, die vorteilhafterweise jene dem ersten Klemmaktor nächste Position seines möglichen Verschiebungsweges ist. Beispielsweise kann sich der Klemmaktor hierbei ganz links befinden. Der zweite Klemmaktor wird in einer zweiten Ausgangsposition, also der Ausgangsposition des zweiten Klemmaktors, aktuiert, so dass er das Objekt greift.
4) Der zweite Bewegungsaktor bewegt nun den zweiten Klemmaktor in die Transportrichtung, beispielsweise nach rechts, also in Richtung vom ersten Klemmaktor weg. Der erste Bewegungsaktor bewegt außerdem den ersten Klemmaktor entgegen der Transportrichtung, z. B. nach links, in die erste Ausgangsposition zurück. Vorteilhafterweise kann der erste Bewegungsaktor den ersten Klemmaktor gleichzeitig mit der Bewegung des zweiten Klemmaktors durch den zweiten Bewegungsaktor bewegen. Die Bewegung des zweiten Bewegungsaktors kann mit der Bewegung des ersten Bewegungsaktors in Schritt 2) auch überlappen, so dass die Beendigung der Aktuierung des ersten Klemmaktors in Schritt 3) nach Beginn der Aktuierung des zweiten Klemmaktors in Schritt 3) erfolgt.
5) Der zweite Klemmaktor beendet dann seine Aktuierung, so dass er das längliche Objekt freigibt und wird, zumindest sofern ein weiterer Durchgang erfolgt, durch den zweiten Bewegungsaktor in die zweite Ausgangsposition, also in seine dem ersten Klemmaktor nächste Position zurückbewegt. Es kann sich nun ein weiterer Durchgang der Schritte 1) bis 5) anschließen, wobei vorteilhaft das Zurückbewegen des zweiten Klemmaktors in Schritt 5) gleichzeitig erfolgen kann mit dem Bewegen des ersten Klemmaktors in Schritt 1).

In ähnlicher Weise kann optional auch eine kontinuierliche Rotation des länglichen Objekts bewirkt werden. Hierbei kann dann zum Beispiel wie folgt vorgegangen werden:
1) Der erste Klemmaktor wird in einer ersten Ausgangswinkelposition aktuiert, so dass er das längliche Objekt greift. Die Ausgangswinkelposition kann dabei eine Extremposition seines Winkelbereichs sein, in dem er bewegbar ist.
2) Ein erster Drehaktor dreht dann den ersten Klemmaktor um die Längsachse des Aufnahmebereichs bzw. des länglichen Objekts in eine gewünschte Drehrichtung. Die gewünschte Drehrichtung ist dabei jene Richtung, in der das längliche Objekt als Ganzes gedreht werden soll. Der erste Drehaktor dreht dabei den ersten Klemmaktor vorzugsweise um einen maximalen Winkel von der ersten Winkelausgangsposition weg, d.h so weit, wie er bauartbedingt drehen kann.
3) Der erste Klemmaktor beendet dann sein Aktuierung, so dass er das längliche Objekt freigibt. Der zweite Klemmaktor wird in einer zweiten Ausgangswinkelposition aktuiert, die vorzugsweise die gleiche Richtung wie die erste Ausgangwinkelposition ist.
4) Ein zweiter Drehaktor, mit dem der zweite Klemmaktor drehbar ist, dreht dann den zweiten Klemmaktor in der gewünschten Drehrichtung, wieder vorzugsweise um einen maximalen Winkel, um den der Drehaktor drehen kann. Der erste Drehaktor dreht außerdem den ersten Klemmaktor entgegen der gewünschten Drehrichtung in die erste Ausgangswinkelposition zurück. Dies kann vorteilhafterweise gleichzeitig mit der Drehung des zweiten Drehaktors erfolgen. Die Drehung des zweiten Drehaktors kann auch über einen Teil des Drehwinkels mit der Drehung des ersten Drehaktors in Schritt 2) überlappen, so dass der zweite Drehaktor aktiviert wird, bevor der erste Drehaktor seine Aktuierung beendet.
5) Der zweite Klemmaktor beendet dann seine Aktuierung, so dass er das längliche Objekt freigibt und wird, zumindest sofern ein weiterer Durchgang erfolgt, durch den zweiten Drehaktor in die zweite Ausgangswinkelposition zurückbewegt. Es kann sich nun ein weiterer Durchgang der Schritte 1) bis 5) anschließen, wobei das Zurückdrehen des zweiten Klemmaktors in Schritt 5) gleichzeitig erfolgen kann mit dem Bewegen des ersten Klemmaktors in Schritt 1).

Im Folgenden soll die Erfindung anhand einiger Figuren beispielhaft erläutert werden. Gleiche Bezugszeichen kennzeichnen dabei gleiche oder entsprechende Merkmale. Die in den Beispielen beschriebenen Merkmale können auch unabhängig vom Beispiel realisiert sein und unter den Beispielen kombiniert werden.

Es zeigt:
- Fig. 1: eine schematische Darstellung eines allgemeinen Beispiels eines erfindungsgemäßen Klemmaktors,
- Fig. 2: eine schematische Darstellung eines erfindungsgemäßen Klemmaktors mit Bewegungsaktoren,
- Fig. 3: eine besonders vorteilhafte Ausgestaltung eines erfindungsgemäßen Klemmaktors,
- Fig. 4: die Seitenansicht eines erfindungsgemäßen Bewegungsmechanismus,
- Fig. 5: eine perspektivische Ansicht des in Fig. 4 gezeigten Bewegungsmechanismus,
- Fig. 6: Positionen zweier Klemmaktoren während der Durchführung eines erfindungsgemäßen Verfahrens mit schematisch dargestellten Bewegungsaktoren,
- Fig. 7: eine beispielhafte Realisierung von Bewegungsaktoren mit Schrittmotoren und Seilzügen,
- Fig. 8: ein Aktuierungsmuster zur Translation eines länglichen Objektes,
- Fig. 9, 10: zwei Ansichten eines Ausschnitts eines erfindungsgemäßen Bewegungsmechanismus mit zwei Zentrierungselementen.

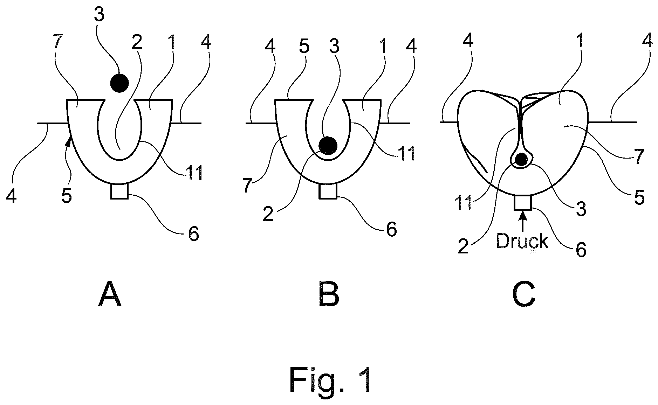

Fig. 1 zeigt schematisch einen erfindungsgemäßen Klemmaktor 1. Dabei zeigt Fig. 1A einen Zustand, bevor ein längliches Objekt 3 in einen Aufnahmebereich 2 des Klemmaktors 1 eingelegt wird, Fig. 1B einen Zustand, in dem das längliche Objekt 3 in den Aufnahmebereich 2 eingelegt ist und Fig. 1C einen Zustand, bei dem ein Fluidvolumen 7 des Klemmaktors mit einem Fluid unter Druck gefüllt wurde, so dass das längliche Objekt 3 durch den Klemmaktor 1 gehalten wird.

Der Klemmaktor 1 weist hier ein u-förmiges Fluidvolumen 7 bzw. einen ein Fluidvolumen enthaltenden Hohlkörper 5 auf. Der Hohlkörper hat eine u-förmige, rinnenförmige oder konkave Aufnahmebereichswand 11, die einen länglichen Aufnahmebereich 2 umläuft und vom Fluidvolumen 7 trennt. Die Aufnahmebereichswand 11 umläuft den Aufnahmebereich 2 über einen Winkel von kleiner als 360° um den Aufnahmebereich 2. Die Aufnahmebereichswand 11 umläuft also den Aufnahmebereich 2 teilweise. Die Aufnahmebereichswand hat eine Form, die in der Figurenebene z.B. ein U beschreibt, und deshalb als z.B. als u-förmig bezeichnet werden kann. Die Schenkel dieses U begrenzen zwischen sich den Aufnahmebereich 2. Die beiden Schenkel der U-Form der Aufnahmebereichswand 11 sind über einen Verbindungsbereich miteinander verbunden, der hier der unterste Bereich der U-Form ist. Es ist dabei nicht entscheidend, wo genau der Verbindungsbereich in die Schenkel übergeht. Die beiden Schenkel sind an ihren dem Verbindungsbereich abgewandten Enden nicht miteinander verbunden, so dass sich nach oben eine Öffnung ergibt, durch die das längliche Objekt 3 in den Aufnahmebereich 2 einlegbar ist.

Die Aufnahmebereichswand wird zumindest bereichsweise durch eine flexible Membran gebildet, die sich, wie in Fig. 1C gezeigt, bei Einführen eines Fluides in das Fluidvolumen 7 aufblähen kann und dann den Aufnahmebereich 2 nach oben, also in Richtung vom Verbindungsbereich weg, verschließt. Auf diese Weise kann das längliche Objekt 3 fest im Aufnahmebereich 2 gehalten werden.

Das Fluidvolumen 7 ist das Innere eines Hohlkörpers 5. Die besagte flexible Membran ist dann Teil der inneren Oberfläche des Hohlkörpers 5, also jener Oberfläche, die dem Aufnahmebereich 2 zugewandt ist. Im in Fig. 1 gezeigten

Beispiel kann die flexible Membran das Fluidvolumen 7 in alle Richtungen begrenzen. Es kann also der Hohlkörper 5 durch die Membran gebildet werden. Der Hohlkörper 5 kann hierbei zum Beispiel als Ballon angesehen werden.

Der Klemmaktor 1 weist außerdem einen Fluidanschluss 6 auf, über den Fluid in das Fluidvolumen 7 einleitbar und aus dem Fluidvolumen 7 auch ableitbar ist. Durch Einleiten von Fluid ist der Klemmaktor 1 in den in Fig. 1C gezeigten Zustand überführbar. Durch Ableiten des Fluids ist er zurück in den in Figuren 1A und 1B gezeigten Zustand überführbar. Der Fluidanschluss 6 ist im gezeigten Beispiel unterhalb des Aufnahmebereichs angeordnet.

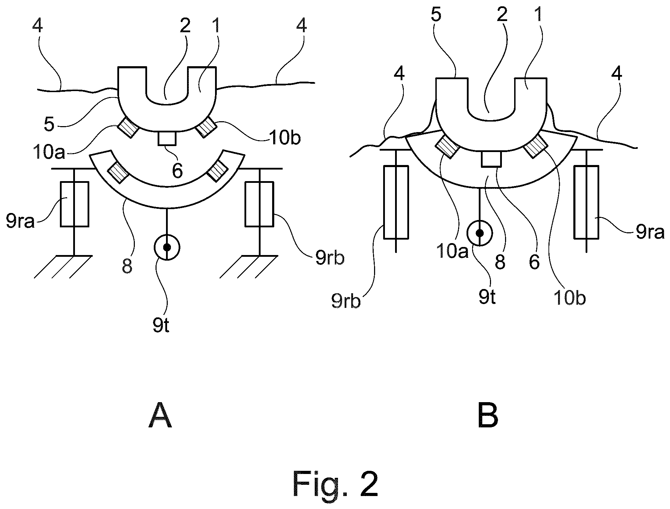

Fig. 2 zeigt schematisch ein Beispiel eines erfindungsgemäßen Bewegungsmechanismus zum Bewegen zumindest eines länglichen Objektes 3. Der Bewegungsmechanismus weist zum einen einen Klemmaktor 1 auf, beispielsweise wie er in Fig. 1 gezeigt ist, sowie zum anderen zumindest einen Bewegungsaktor 9ra, 9rb, 9t, mit dem der Klemmaktor in Richtung einer Längsrichtung des Aufnahmebereichs 2 verschiebbar ist und um den Aufnahmebereich 2 drehbar ist. Der Klemmaktor 1 ist dabei mit den Aktoren 9ra, 9rb, 9t über ein Verbindungselement 8 verbindbar, in das der Klemmaktor 1 einsetzbar ist. Fig. 2A zeigt dabei den Klemmaktor 1 getrennt vom Verbindungselement 8 und Fig. 2B zeigt den Klemmaktor 1 eingesetzt im Verbindungselement 8. Der Klemmaktor 1 weist an seiner dem Aufnahmebereich 2 abgewandten Unterseite hierzu Verbindungsteile 10a, 10b auf, die in entsprechende Ausnehmungen in der Aufnahme 8 eingreifen können, so dass der Klemmaktor 1 durch die Aufnahme 8 gehalten werden kann und dass eine durch die Aufnahme 8 ausgeübte lineare Kraft und ein durch die Aufnahme ausgeübtes Drehmoment auf den Klemmaktor 1 übertragbar sind.

Die Aktoren 9ra, 9rb und 9t sind hier lediglich schematisch zu verstehen. Dabei ist über die Aktoren 9ra und 9rb eine Drehbewegung auf den Klemmaktor 1 ausübbar und über den Aktor 9t eine Translationsbewegung in Richtung der Längsachse des Aufnahmebereichs 2.

In Fig. 2 ist auch der Nutzen der Folie 4 gut zu erkennen. Die Folie 4 umläuft das den Hohlkörper 5 vollständig und erstreckt sich in einer Ebene parallel zur Längsrichtung des Aufnahmebereichs 2. Die Folie 4 deckt alle Elemente unterhalb ab und trennt diese also steril von der Oberseite und insbesondere dem Aufnahmebereich 2. Auf diese Weise wird ein Kontakt von Elementen oberhalb der Folie mit den Aktoren 9ra, 9rb, 9t und weiteren Elementen des Bewegungsmechanismus vermieden und die Sterilität gewährleistet. Insbesondere ermöglicht die Verwendung der Folie 4 die Aufteilung des Bewegungsmechanismus in sterile und unsterile Teile. Der Klemmaktor 1 kann in Verbindung mit der Abdeckfolie 4 steril gefertigt werden, während der Verbindungsmechanismus 8 und die restliche Mechanik unsteril verwendet werden können.

Die Objekte 3, die mit dem erfindungsgemäßen Klemmaktor 1 greifbar sind, können beispielsweise schlauchartige Geräte, insbesondere medizinische endovaskuläre Katheter und/oder Führungsdrähte sein. Der Klemmaktor 1 kann hydraulisch und/oder pneumatisch betrieben werden. In Fig. 2 ist für die Translation nur ein Aktor 9t gezeigt, es können jedoch auch mehrere Aktoren verwendet werden, was insbesondere vorteilhaft ist, wenn mehrere Klemmaktoren 1 in dem Bewegungsmechanismus zum Einsatz kommen. Die Aktoren 9ra, 9rb, 9t können dabei einfach wirkende oder doppelt wirkende Aktoren sein. Es können insbesondere zwei einfach wirkende Aktoren anstelle eines doppelt wirkenden Aktors verwendet werden.

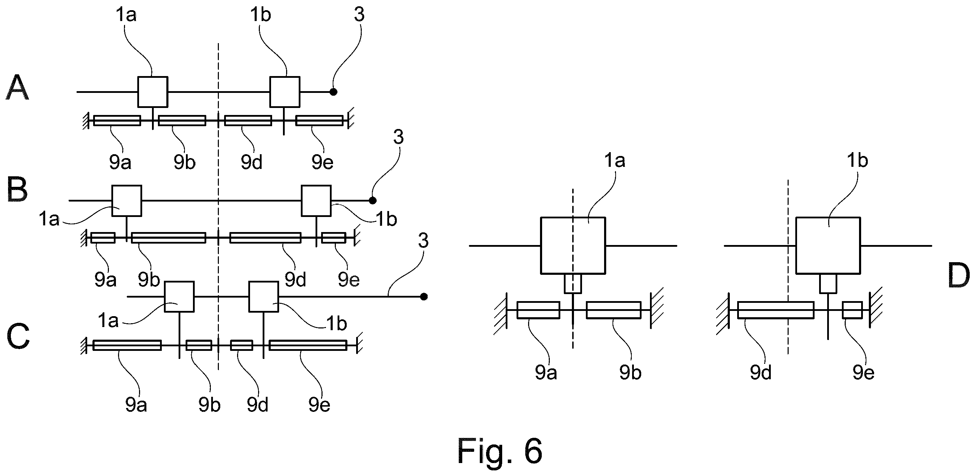

Ein einzelner Klemmaktor 1 ermöglicht relativ kleine Rotationen und/oder Translationen. Werden zwei Klemmaktoren 1 mit zwei Bewegungsaktoren 9ra, 9rb, 9t verbunden und abwechselnd aktuiert und zurückgestellt, so kann eine kontinuierliche Translation und/oder Rotation ermöglicht werden. Ein Bewegungsmechanismus zum Bewegen eines länglichen Objektes wie beispielsweise eines schlauchartigen Gerätes (z.B. Katheter/Führungsdraht) weist also vorzugsweise zumindest zwei Klemmaktoren 1 und zwei Bewegungsaktoren 9ra, 9rb, 9t auf. Eine Translation oder Rotation kann mit drei einfach wirkenden, mit vier einfach wirkenden, mit zwei doppeltwirkenden oder mit zwei einfach und einem doppelt wirkenden Aktoren bewirkt werden. Die Translation mit vier einfach wirkenden Aktoren ist in Fig. 6 beispielhaft gezeigt.

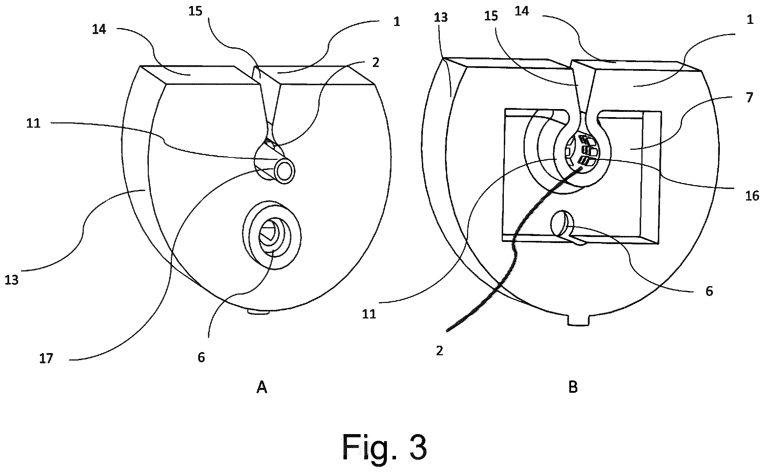

Fig. 3 zeigt beispielhaft einen erfindungsgemäßen Klemmaktor 1, wobei Fig. 3A den Klemmaktor vollständig zeigt, und Fig. 3B einen Schnitt durch den in Fig. 3A gezeigten Klemmaktor zeigt. Der Klemmaktor 1 hat hier eine äußere Form, die einen zylinderförmigen Bereich 13 mit einem Querschnitt in einer Ebene senkrecht zu einer Längsrichtung des Aufnahmebereichs 2 aufweist, der die Form eines Kreisbogens mit einem Enden des Kreisbogens verbindenden geraden Abschnitt hat, wobei die äußere Oberfläche in einem Bereich mit geradem Querschnitt einen zu der Längsrichtung des Aufnahmebereichs 2 parallelen Einschnitt 15 aufweist, in dem die äußere Oberfläche 13 in die Aufnahmebereichswand 11 übergeht. Dabei hat der kreisbogenförmige Querschnitt die Längsachse des Aufnahmebereichs 2 als Mittelachse. Die Enden des kreisbogenförmigen Abschnitts der Oberfläche 13 in Richtung des Kreisbogens sind über einen ebenen Oberflächenabschnitt 14 verbunden. Der ebene Oberflächenabschnitt 14 ist durch einen zur Längsachse des Aufnahmebereichs 2 parallelen Einschnitt 15 unterbrochen, der nach innen, also in Richtung des Aufnahmebereichs 2 in die Aufnahmebereichswand 11 übergeht. Das Fluidvolumen 7 ist im Inneren des Hohlkörpers 5 ausgebildet. Es hat in einer Ebene senkrecht zur Längsrichtung des Aufnahmebereichs 2 einen rechteckigen oder quadratischen Querschnitt, in den aus Richtung des ebenen Oberflächenabschnitts 14 der Oberfläche des Hohlkörpers der Aufnahmebereich 2 und die Aufnahmebereichswand 11 hineinragt.

Die Aufnahmebereichswand 11 weist auf ihrer dem Aufnahmebereich 2 zugewandten Seite Noppen 16 auf, die einen Halt eines länglichen Objektes im Aufnahmebereich verbessern. In Fig. 3A ist ein Katheter 17 als Beispiel eines länglichen Objektes im Aufnahmebereich 2 gezeigt.

Der Fluidanschluss 6 ist hier ein parallel zur Längsachse des Aufnahmebereichs verlaufendes Rohr, dass in das Fluidvolumen 7 mündet.

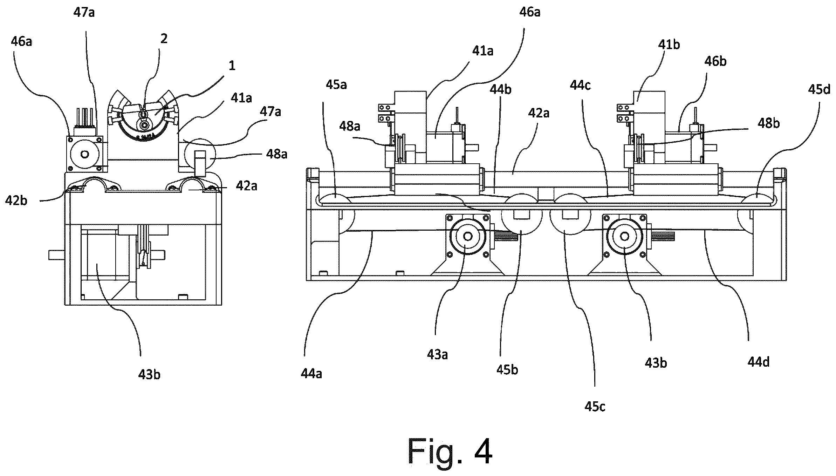

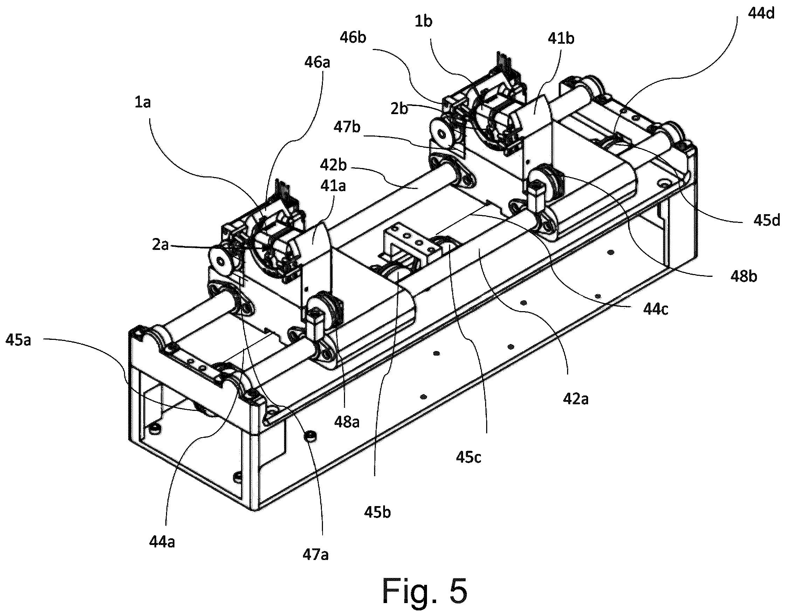

Fig. 4 und Fig. 5 zeigen eine Ausgestaltung eines erfindungsgemäßen Bewegungsmechanismus mit zwei Klemmaktoren 1, die in beweglichen Elementen 41a und 41b angeordnet sind. Die beweglichen Elemente 41a und 41b sind auf Stangen 42a und 42b verschiebbar gelagert und werden zur Bewegung entlang der Längsrichtungen der Stangen 42a und 42b aktuiert. Die Aktuierung erfolgt hier mittels Schrittmotoren 43a und 43b, die über Seilzüge 44a, 44b, 44c, 44d an den beweglichen Elementen 41a und 41b angreifen. Die Seilzüge werden dazu über Umlenkrollen 45a, 45b, 45c, 45d vom entsprechenden Motor 43a, 43b zu den beweglichen Elementen 41a, 41b geführt. Dabei läuft der Seilzug 44a vom Schrittmotor 43a über die Rolle 45a zu einem linken Angriffspunkt am beweglichen Element 41a und der Seilzug 44b vom Schrittmotor 43a über die Rolle 45b zu einem rechten Angriffspunkt am beweglichen Element 41a. Der Seilzug 44c verläuft vom Schrittmotor 43b über die Rolle 45c zu einem linken Angriffspunkt am beweglichen Element 41b und der Seilzug 44d verläuft vom Schrittmotor 43b über die Rolle 45d zu einem rechten Angriffspunkt am beweglichen Element 41b. Die Seilzüge 44a bis 44d sind dabei parallel zur Richtung der Verschiebung der beweglichen Elemente 41a und 41b, das heißt parallel zu den Stangen 42a, 42b, geführt.

Die beweglichen Elemente 41a und 41b weisen außerdem jeweils einen Aktor 46a, 46b, hier Schrittmotoren, zum Drehen des entsprechenden Klemmaktors 1a, 1b um seinen Aufnahmebereich auf. Dabei greift der Aktor 46a, 46b jeweils über einen Seilzug 47a, 47b an einem Drehelement an, in dem der entsprechende Klemmaktor 1a, 1b angeordnet ist. Die Seilzüge 47a, 47b sind dabei über eine Rolle des entsprechenden Aktors 46a, 46b geführt und außerdem über jeweils eine Rolle 48a, 48b, so dass die Seilzüge 47a, 47a an den Drehelementen von gegenüberliegenden Seiten angreifen. Eine Drehung des Aktors 46a, 46b führt so zu einer Drehung des entsprechenden Drehelements und damit des Klemmaktors 1a, 1b.

Im gezeigten Beispiel liegen die Längsrichtungen der Aufnahmebereiche 2a, 2b koaxial zueinander. Die Klemmaktoren 1a, 1b sind wie in Fig. 3 gezeigt ausgestaltet.

Fig. 6 zeigt schematisch die Anordnung von Bewegungsaktoren zum Verschieben der Klemmaktoren 1a, 1b in den Figuren 4 und 5. Dabei sind in den Figuren 6A, 6B und 6C vier Bewegungsaktoren 9a, 9b, 9d, 9e vorgesehen, die gemeinsam die Bewegung der Klemmaktoren 1a und 1b bewirken. Da sie eine Translation bewirken entsprechen sie dem Aktor 9t in Figur 2. In den Figuren 6A bis 6C können zwei Module identifiziert werden, die durch eine gestrichelte Linie getrennt dargestellt sind. Alternativ kann die Translation auch mit drei einfach wirkenden anstelle von vier einfach wirkenden Aktoren bewirkt werden. Dabei werden die beiden Module jeweils mit einem Aktor mit der Umgebung (links und rechts) verbunden und zwischen den beiden Modulen wäre ein dritter Aktor vorgesehen, der die beiden Module miteinander verbindet. In den Figuren 6A bis 6C würden also die Aktoren 9b und 9d durch einen einzelnen Aktor ersetzt. Auch mit zwei doppelt wirkenden Aktoren kann die Translation bewirkt werden. Es würden dann die Aktoren 9a/9b und 9d/9e jeweils durch einen doppelt wirkenden Aktor ersetzt, der dann jeweils mit der Umgebung verbunden würde.

Fig. 6D zeigt außerdem eine Ausgestaltung, wobei die Translation der Klemmaktoren 1a und 1b jeweils durch zwei Aktoren 9a, 9b bzw. 9d, 9e bewirkt wird. Die Aktoren 9b und 9d sind also hier nicht miteinander verbunden. Auf diese Weise können die Klemmaktoren 1a und 1b in großem Abstand voneinander angeordnet werden und komplexere Bewegungsprofile realisiert werden, da die Klemmaktoren unabhängig voneinander bewegt werden können.

Mit den in Fig. 6 gezeigten Aktoren 9a, 9b, 9d, 9e kann eine Translation des länglichen Objektes 3 bewirkt werden. Dabei greift zunächst der erste Klemmaktor 1a das längliche Objekt 3 in einer ersten Ausgangsposition. Es bewegen dann die Bewegungsaktoren 9a und 9b den ersten Klemmaktor 1a in die Transportrichtung, in Fig. 6 nach rechts. Die erste Ausgangsposition kann beispielsweise die Position sein, in der der Klemmaktor 1a in Fig. 6B gezeigt ist.

Ist der erste Klemmaktor 1a in der Endposition, beispielsweise in der in Fig. 6C gezeigten Position, angekommen, so beendet er seine Aktuierung, so dass er das längliche Objekt 3 freigibt. Der zweite Klemmaktor 1b wird dann in einer zweiten Ausgangsposition aktuiert, beispielsweise der in Fig. 6C gezeigten Position, wo er sich ganz links bzw. in seiner dem ersten Klemmaktor 1a nächsten Position, befindet. Der zweite Klemmaktor 1b greift dann das längliche Objekt 3. Anschließend bewegen die Bewegungsaktoren 9d und 9e den zweiten Klemmaktor 1b in der Transportrichtung, also in Fig. 6 nach rechts, während der erste Klemmaktor 1a entgegen der Transportrichtung in die erste Ausgangsposition zurückbewegt wird. Dieser Schritt führt zu der in Fig. 6B gezeigten Situation. Der zweite Klemmaktor 1b kann dann seine Aktuierung beenden, so dass er das längliche Objekt 3 freigibt. Er kann dann in seine zweite Ausgangsposition zurückbewegt werden.

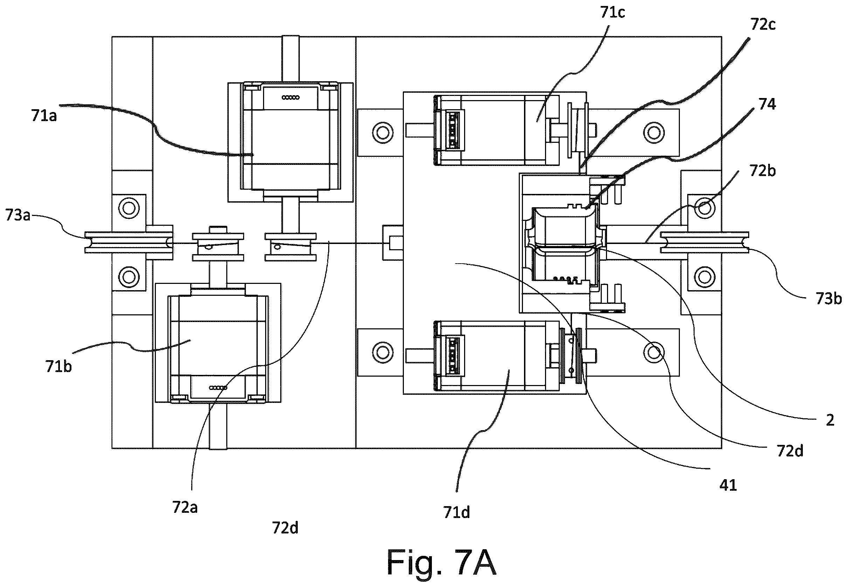

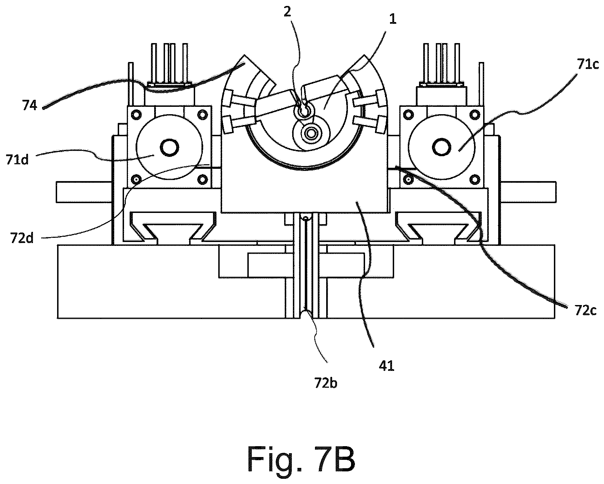

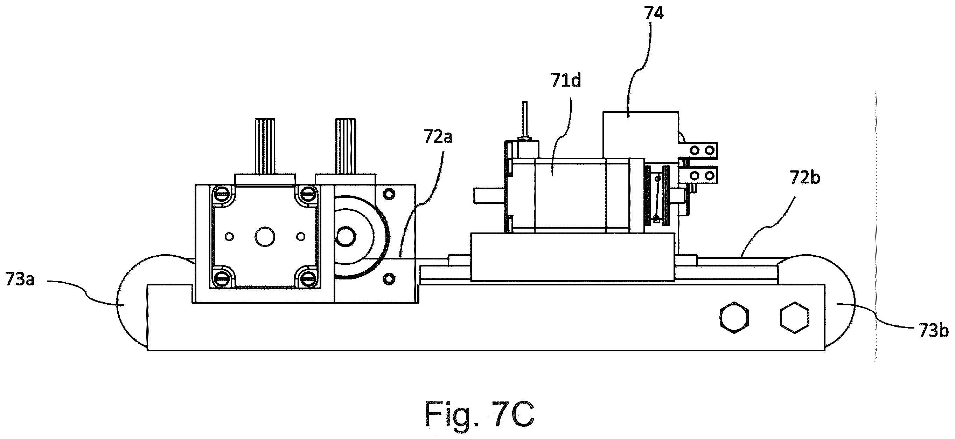

Die Bewegungsaktoren können beispielsweise mittels eines Seilmechanismus realisiert werden, der durch Schrittmotoren angetrieben wird. Ein Beispiel hierfür zeigen die Figuren 7A, 7B und 7C. Dabei zeigt die Fig. 7A das Beispiel einer Draufsicht, die Fig. 7B das Beispiel mit Blickrichtung in Richtung der Längsachse des Aufnahmebereichs 2 und Fig. 7C den Mechanismus in seiner seitlichen Ansicht senkrecht zur Längsrichtung des Aufnahmebereichs 2. Wie in Fig. 7A zu erkennen ist, weist die Vorrichtung zum Antrieb eines der Klemmaktoren 1 vier Motoren 71a, 71b, 71c, 71d, hier Schrittmotoren, auf. Die Schrittmotoren 71a und 71b greifen über Seilzüge 72a und 72b an dem Bewegungselement 41 an und können dies in Richtung der Längsachse des Aufnahmebereichs 2 verschieben. Dabei wird einer der Seilzüge 72b über Rollen 73a und 73b zu dem Ende des Schlittens 41 geführt, das dem Seilzug 72a gegenüber liegt. Der Seilzug 72a wird durch den Motor 71a gezogen und der Seilzug 72b wird durch den Motor 71b gezogen.

Der Klemmaktor 1 ist in einem Drehelement 74 angeordnet, das um die Längsachse des Aufnahmebereichs 2 drehbar ist. Die Drehung des Drehelements 74 und damit des Klemmaktors 1 ist mittels der Motoren 71c und 71d realisiert. Dazu ist der Motor 71c über einen Seilzug 72c mit dem Drehelement 74 an dessen Umfang verbunden und der Motor 71d ist über einen Seilzug 72d mit dem Drehelement 74 an dessen Umfang verbunden. Durch Ausübung von Zugkraft durch die Motoren 71c und 71d ist daher das Drehelement 74 um die Längsachse des Aufnahmebereichs 2 drehbar.

Die Motoren 71a bis 71d weisen zum Aufrollen der entsprechenden Seilzüge 72a bis 72d jeweils Rollen auf, die koaxial sind zur Drehachse des entsprechenden Motors 71a bis 71d.

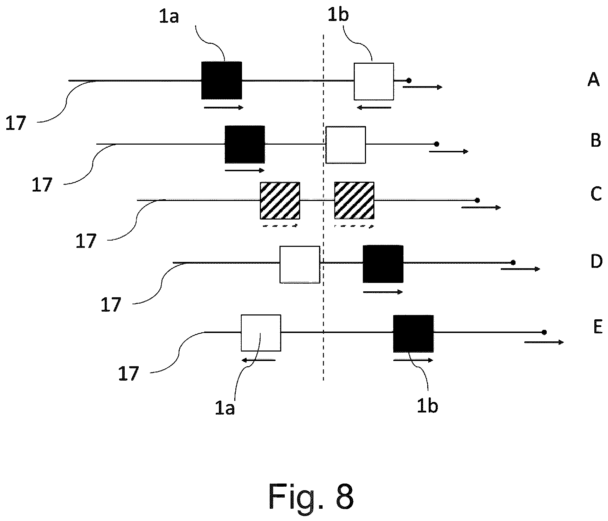

Figur 8 zeigt beispielhaft ein Aktuierungsmuster, wie es zum Beispiel mit der Anordnung in den Figuren 4 und 5 ausführbar ist. Dabei kennzeichnet ein schwarz ausgefülltes Quadrat einen aktuierten Klemmaktor 1a, 1b und ein weißes Quadrat einen nicht aktuierten Klemmaktor 1a, 1b. Schraffiere Quadrate zeigen einen Zustand, in dem beide Aktoren 1a und 1b gemeinsam aktuiert sind. Pfeile geben die Bewegungsrichtung der Aktoren 1a, 1b und des länglichen Objektes 17 an. Die Figuren 8A bis 8E zeigen dabei aufeinanderfolgende Schritte eines Verfahrens, bei dem das längliche Objekt nach rechts bewegt wird. Es werden im Einzelnen folgende Schritte ausgeführt, wobei die Buchstaben den Teilbildern der Figur 8 entsprechen:
A) Der erste Klemmaktor 1a wird in einer ersten Ausgangsposition aktuiert, so dass er das längliche Objekt 17 greift. Die Ausgangsposition kann dabei eine Extremposition seines Bewegungsbereichs sein. Zum Beispiel kann der erste Klemmaktor 1a sich ganz links befinden. Der erste Bewegungsaktor bewegt den ersten Klemmaktor 1a in eine Transportrichtung, hier nach rechts, die auf den zweiten Klemmaktor 1b hin gerichtet ist. Während sich der Klemmaktor 1a nach rechts bewegt, bewegt der Klemmaktor 1a sich nach links in eine zweite Ausgangsposition, sofern er sich rechts befindet.
B) Der zweite Klemmaktor 1b liegt in diesem Teilbild in der zweiten Ausgangsposition vor, währen sich der erste Klemmaktor 1a weiter nach rechts bewegt.
C) Noch während sich der erste Klemmaktor nach rechts bewegt, startet der zweite Klemmaktor seine Bewegung nach rechts. Hierbei können die Klemmaktoren 1a und 1b für eine Zeit lang beide aktuiert sein, was durch die Schraffur angedeutet ist.
D) Der erste Klemmaktor 1a beendet dann seine Aktuierung. Der zweite Klemmaktor 1b ist aktuiert und bewegt sich nach rechts. Dadurch transportiert er das Objekt 17 nach rechts.
E) Der erste Klemmaktor 1a wird während der Bewegung des zweiten Klemmaktors 1b in die erste Ausgangsposition nach links zurückbewegt. Er ist dabei nicht aktuiert.

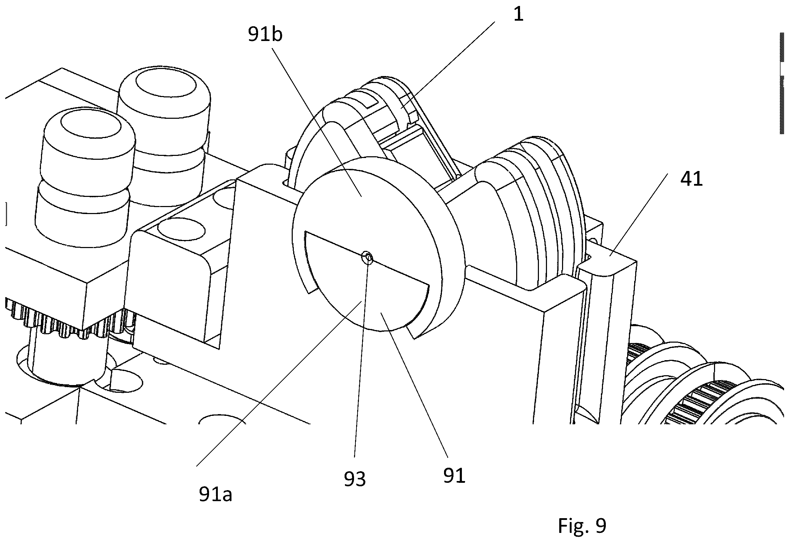

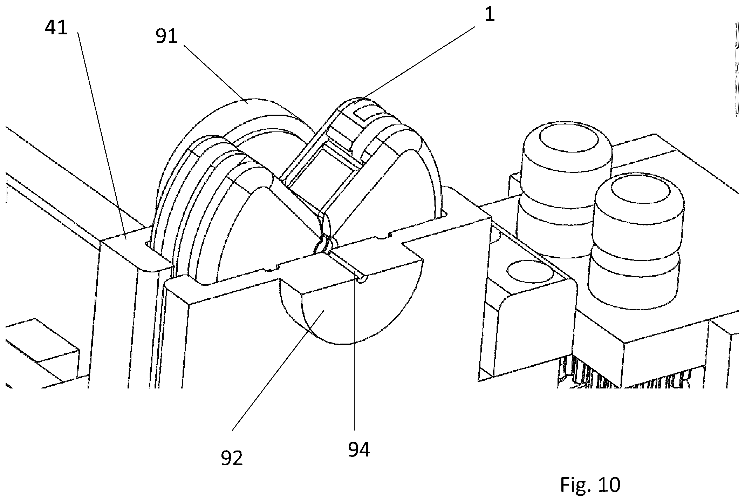

Figur 9 und 10 zeigen einen Teil eines erfindungsgemäßen Bewegungsmechanismus mit zwei Zentrierungselementen 91, 92, die auf gegenüberliegenden Seiten des Aufnahmebereichs des Klemmaktors 1 angeordnet sind. Das Zentrierungselement 91 weist dabei zwei Teile 91a und 91b auf, die zusammensetzbar sind und zwischen sich eine Durchgangsbohrung 93 ausbilden. In dieser kann das längliche Objekt zentriert werden. Das Zentrierungselement 92 weist eine Rinne 94 auf, in die das längliche Objekt eingelegt werden kann wodurch es in der Rinne 94 zentriert wird. Die Durchgangsbohrung 93 und die Rinne 94 verlaufen koaxial zur Mittelachse des Aufnahmebereichs des Klemmaktors 1. Wird der Klemmaktor 1 zu einer Drehung um die Mittelachse aktuiert, so kann durch die Zentrierungselemente gewährleistet werden, dass sich das längliche Objekt auf der Drehachse befindet und bei der Drehung nicht versetzt wird.

## Patentansprüche

1. Bewegungsmechanismus zum Bewegen zumindest eines länglichen Objekts (3, 17),
wobei der Bewegungsmechanismus zumindest einen Klemmaktor (1) zum Halten eines länglichen Objektes (3, 17) aufweist,
wobei der Klemmaktor (1)
einen Hohlkörper (5) mit einem Fluidvolumen (7) aufweist,
wobei der Hohlkörper (5) eine u-förmige, rinnenförmige oder konkave Aufnahmebereichswand (11) aufweist, die einen länglichen Aufnahmebereich (2) zumindest teilweise umläuft, in den das längliche Objekt (3, 17) einlegbar ist,
wobei die Aufnahmebereichswand (11) den Aufnahmebereich (2) vom Fluidvolumen (7) trennt,
wobei die Aufnahmebereichswand (11) durch eine flexible Membran gebildet wird,
wobei der Klemmaktor (1) außerdem einen Fluidanschluss (6) aufweist, über den Fluid in das Fluidvolumen (7) einleitbar und aus dem Fluidvolumen (7) ableitbar ist, und
wobei der Bewegungsmechanismus außerdem zumindest einen Bewegungsaktor (9ra, 9rb, 9t) aufweist, mit dem der Klemmaktor (1) in Richtung einer Längsrichtung des Aufnahmebereichs (2) verschiebbar und/oder um den Aufnahmebereich (2) drehbar ist,
wobei der Bewegungsmechanismus gemäß Variante A zumindest einen ersten und einen zweiten der Klemmaktoren (1a, 1b) aufweist, wobei der ersten Klemmaktor (1a) an einem ersten Bewegungsaktor (9a, 9b) angeordnet ist und der zweite Klemmaktor (1b) an einem zweiten Bewegungsaktor (9d, 9e) angeordnet ist,
wobei die Klemmaktoren (1a, 1b) mit den Bewegungsaktoren (9a, 9b, 9d, 9e) in Richtung der Längsrichtung ihrer Aufnahmebereiche (2a, 2b) verschiebbar sind,
wobei der Bewegungsmechanismus ausgestaltet ist zum Durchführen des nachfolgenden Verfahrens: wobei die Klemmaktoren (1a, 1b) und Bewegungsaktoren (9a, 9d, 9b, 9e) nach Einlegen des länglichen Objects (3, 17) in die Aufnahmebereiche (2a, 2b) beider Klemmaktoren (1a, 1b) in einem Aktuierungsmuster aktuiert werden,
wobei das Aktuierungsmuster wiederholt folgende Schritte umfasst, die auch überlappen können:
1) der erste Klemmaktor (1a) wird in einer ersten Ausgangsposition aktuiert, so dass er das längliche Objekt (3, 17) greift,
2) der erste Bewegungsaktor (9a, 9b) bewegt den ersten Klemmaktor (1a) in eine Transportrichtung,
3) der erste Klemmaktor (1a) beendet seine Aktuierung, so dass er das längliche Objekt (3, 17) freigibt, und der zweite Klemmaktor (1b) wird in einer zweiten Ausgangsposition aktuiert, so dass er das Objekt (3, 17) greift,
4) der zweite Bewegungsaktor (9d, 9e) bewegt den zweiten Klemmaktor (1b) in die Transportrichtung und der erste Bewegungsaktor (9a, 9b) bewegt den ersten Klemmaktor (1a) entgegen der Transportrichtung in die erste Ausgangsposition zurück,
5) der zweite Klemmaktor (1b) beendet seine Aktuierung, so dass er das längliche Objekt (3, 17) freigibt und wird durch den zweiten Bewegungsaktor (9d, 9e) in die zweite Ausgangsposition bewegt,
und/oder
wobei beim Bewegungsmechanismus gemäß Variante B der erste Klemmaktor (1a) an einem ersten Drehaktor angeordnet ist und der zweite Klemmaktor (1b) an einem zweiten Drehaktor angeordnet ist,
wobei die Klemmaktoren (1a, 1b) mit den jeweiligen Drehaktoren um die Längsachse ihrer Aufnahmebereiche (2a, 2b) drehbar sind,
wobei der Bewegungsmechanismus ausgestaltet ist zum Durchführen des nachfolgenden Verfahrens:
wobei die Klemmaktoren (1a, 1b) nach Einlegen des länglichen Objects (3, 17) in die Aufnahmebereiche (2a, 2b) beider Klemmaktoren (1a, 1b) und Drehaktoren in einem Aktuierungsmuster aktuiert werden,
wobei das Aktuierungsmuster wiederholt folgende Schritte umfasst,
die auch überlappen können:
1) der erste Klemmaktor (1a) wird in einer ersten Ausgangswinkelposition aktuiert, so dass er das längliche Objekt (3, 17) greift,
2) der erste Drehaktor dreht dann den ersten Klemmaktor (1a) um die Längsachse des Aufnahmebereichs (2a) oder des länglichen Objekts (3, 17) in eine gewünschte Drehrichtung,
3) der erste Klemmaktor (1a) beendet dann sein Aktuierung, so dass er das längliche Objekt (3, 17) freigibt, und der zweite Klemmaktor (1b) wird in einer zweiten Ausgangswinkelposition aktuiert,
4) der zweite Drehaktor dreht dann den zweiten Klemmaktor (1b) in der gewünschten Drehrichtung, und der erste Drehaktor dreht außerdem den ersten Klemmaktor (1a) entgegen der gewünschten Drehrichtung in die erste Ausgangswinkelposition zurück,
5) der zweite Klemmaktor (1b) beendet dann seine Aktuierung, so dass er das längliche Objekt (3, 17) freigibt und wird, zumindest sofern ein weiterer Durchgang erfolgt, durch den zweiten Drehaktor in die zweite Ausgangswinkelposition zurückbewegt.

2. Bewegungsmechanismus nach dem vorhergehenden Anspruch, wobei die flexible Membran alle Seiten des Fluidvolumens (7) begrenzt, und/oder
wobei sich der Aufnahmebereich (2) entlang einer Geraden erstreckt.

3. Bewegungsmechanismus nach einem der vorhergehenden Ansprüche, wobei die flexible Membran in einem Zustand, in dem keine äußeren Kräfte auf sie wirken, eine Form hat, die durch Verschiebung einer geschlossenen ebenen Kurve entlang eines um eine Mittelachse gekrümmten Verschiebewegs entsteht, wobei die Mittelachse an jeder Position der Verschiebung außerhalb der von der Kurve umschlossenen Fläche und in der Ebene der Kurve liegt,
wobei Enden des Verschiebungswegs einen Winkel größer als Null um die Mittelachse einschließen,
wobei vorzugsweise der Aufnahmebereich länglich entlang der Mittelachse verläuft,
wobei vorzugsweise der Verschiebeweg die Mittelachse um mehr als 180 Grad, vorzugsweise um mehr als 270 Grad umläuft.

4. Bewegungsmechanismus nach einem der vorhergehenden Ansprüche, wobei die Aufnahmebereichswand (11) den Aufnahmebereich (2) über einen Winkel von mehr als 180° umläuft.

5. Bewegungsmechanismus nach einem der vorhergehenden Ansprüche, wobei der Hohlkörper (5) über die Aufnahmebereichswand (11) verbundene Oberflächenabschnitte aufweist, die einen spitzen Winkel um den Aufnahmebereich (2) einschließen.

6. Bewegungsmechanismus nach einem der vorhergehenden Ansprüche, wobei dem Aufnahmebereich (2) zugewandte Seiten des Fluidvolumens (7) und dem Aufnahmebereich (2) abgewandte Seiten des Fluidvolumens (7) parallel zueinander verlaufen.

7. Bewegungsmechanismus nach einem der vorhergehenden Ansprüche, wobei der Hohlkörper (5) eine äußere Oberfläche aufweist, die einen zylinderförmigen Bereich (13) mit einem Querschnitt in einer Ebene senkrecht zu einer Längsrichtung des Aufnahmebereichs (2) aufweist, der die Form eines Kreisbogens mit einem Enden des Kreisbogens verbindenden geraden Abschnitt hat oder der eine rechteckige Form hat, wobei die äußere Oberfläche in einem Bereich mit geradem Querschnitt einen zu der Längsrichtung des Aufnahmebereichs (2) parallelen Einschnitt (15) aufweist, in dem die äußere Oberfläche (13) in die Aufnahmebereichswand (11) übergeht.

8. Bewegungsmechanismus nach einem der vorhergehenden Ansprüche, aufweisend eine Folie (4), die den Hohlkörper (5), vorzugsweise unterhalb des Aufnahmebereichs (2), vollständig umläuft und die sich in einer Ebene parallel zur Längsrichtung des Aufnahmebereichs (2) erstreckt.

9. Bewegungsmechanismus nach einem der vorhergehenden Ansprüche, wobei die Aufnahmebereichswand (11) in einem Schnitt in einer Ebene senkrecht zu einer Richtung einer Erstreckung des Aufnahmebereichs (2) die Form eines zweidimensionalen U hat.

10. Bewegungsmechanismus nach einem der vorhergehenden Ansprüche, weiter aufweisend zumindest ein Zentrierungselement (91, 92), das auf zumindest einer Seite des Aufnahmebereichs (2) in Richtung dessen Längsrichtung angeordnet ist, wobei mit dem zumindest einen Zentrierungselement (91, 92) das längliche Objekt (3, 17) auf eine Mittelachse des Aufnahmebereichs (2) zentrierbar ist, wobei vorzugsweise das zumindest eine Zentrierungselement (91, 92) eine Rinne (94) oder eine Durchgangsborung (93) aufweist, die koaxial zu der Mittelachse des Aufnahmebereichs (2) verläuft.

11. Bewegungsmechanismus nach einem der vorhergehenden Ansprüche, aufweisend zumindest zwei der Klemmaktoren (1a, 1b), deren Aufnahmebereiche (2a, 2b) koaxial zueinander liegen,
weiter aufweisend zumindest einen der Bewegungsaktoren (9a, 9b, 9d, 9e) für jeden der Klemmaktoren (1a, 1b).

12. Bewegungsmechanismus nach einem der vorhergehenden Ansprüche, wobei der Bewegungsmechanismus eine u-förmige, rinnenförmige oder konkave Aufnahme aufweist,
wobei der Klemmaktor zwischen Schenkeln der Aufnahme angeordnet ist,
wobei eine Achse, um die die Aufnahme verläuft, parallel liegt zu einer Achse um die Aufnahmebereichswand (11) verläuft.

## Claims

1. A movement mechanism for moving at least one elongated object (3, 17),
the movement mechanism comprising at least one clamping actuator (1) to hold an elongated object (3, 17),
the clamping actuator (1)
having a hollow body (5) with a fluid volume (7),
the hollow body (5) having a U-shaped, channel-shaped, or concave receiving area wall (11) that at least partially surrounds an elongated receiving area (2) into which the elongated object (3, 17) can be inserted,
the receiving area wall (11) separating the receiving area (2) from the fluid volume (7),
the receiving area wall (11) being formed by a flexible membrane,
the clamping actuator (1) also having a fluid connection (6) through which fluid can be introduced into the fluid volume (7) and discharged from the fluid volume (7), and
the movement mechanism also comprising at least one movement actuator (9ra, 9rb, 9t) to move the clamping actuator (1) in the longitudinal direction of the receiving area (2) and/or rotate the clamping actuator (1) around the receiving area (2),
the movement mechanism according to variant A having at least a first and a second of the clamping actuators (1a, 1b),
the first clamping actuator (1a) being arranged on a first movement actuator (9a, 9b) and the second clamping actuator (1b) being arranged on a second movement actuator (9d, 9e),
the clamping actuators (1a, 1b) being displaceable with the movement actuators (9a, 9b, 9d, 9e) towards the longitudinal direction of their receiving areas (2a, 2b),
the movement mechanism being designed to perform the following process: the clamping actuators (1a, 1b) and movement actuators (9a, 9d, 9b, 9e) being actuated in an actuation pattern after the elongated object (3, 17) has been inserted into the receiving areas (2a, 2b) of both clamping actuators (1a, 1b),
the actuation pattern repeatedly comprising the following steps, which may also overlap:
1) the first clamping actuator (1a) is actuated in a first initial position so that it grips the elongated object (3, 17),
2) the first movement actuator (9a, 9b) moves the first clamping actuator (1a) in a transport direction,
3) the first clamping actuator (1a) terminates its actuation, thereby releasing the elongated object (3, 17), and the second clamping actuator (1b) is actuated in a second initial position, thereby gripping the object (3, 17),
4) the second movement actuator (9d, 9e) moves the second clamping actuator (1b) in the transport direction and the first movement actuator (9a, 9b) moves the first clamping actuator (1a) back to the first initial position against the transport direction,
5) the second clamping actuator (1b) terminates its actuation, thereby releasing the elongated object (3, 17), and is moved into the second initial position by the second movement actuator (9d, 9e),
and/or
the first clamping actuator (1a) in the movement mechanism according to variant B being arranged on a first rotating actuator and the second clamping actuator (1b) being arranged on a second rotating actuator, the clamping actuators (1a, 1b) being rotatable with the respective rotating actuators around the longitudinal axis of their receiving areas (2a, 2b), the movement mechanism being designed to perform the following method:
the clamping actuators (1a, 1b) being actuated in a actuation pattern after the elongated object (3, 17) has been inserted into the receiving areas (2a, 2b) of both clamping actuators (1a, 1b) and rotating actuators, the wake pattern repeatedly comprising the following steps, which may also overlap:
1) the first clamping actuator (1a) is actuated in a first initial angular position so that it grips the elongated object (3, 17),
2) the first rotating actuator then rotates the first clamping actuator (1a) around the longitudinal axis of the receiving area (2a) or the elongated object (3, 17) in a desired direction of rotation,
3) the first clamping actuator (1a) terminates its actuation, thereby releasing the elongated object (3, 17), and the second clamping actuator (1b) is actuated in a second initial angular position,
4) the second rotating actuator then rotates the second clamping actuator (1b) in the desired direction of rotation, and the first rotating actuator also rotates the first clamping actuator (1a) back to the first initial angular position in the opposite direction to the desired direction of rotation,
5) the second clamping actuator (1b) terminates its actuation, thereby releasing the elongated object (3, 17), and, at least if another pass is made, is moved back to the second initial angular position by the second rotating actuator.

2. The movement mechanism according to the preceding claim, the flexible membrane delimiting all sides of the fluid volume (7), and/or
the receiving area (2) extending along a straight line.

3. The movement mechanism according to any one of the preceding claims, the flexible membrane, in a state in which no external forces act on it, having a shape that is created by moving a closed plane curve along a shifting path curved around a central axis, the centre axis lying outside the area enclosed by the curve and in the plane curve at every position of displacement,
ends of the shifting path enclose an angle greater than zero around the center axis,
the receiving area extending preferably longitudinally along the central axis,
the shifting path rotating preferably around the central axis by more than 180 degrees, preferably by more than 270 degrees.

4. The movement mechanism according to any one of the preceding claims, the receiving area (11) surrounding the receiving area (2) over an angle of more than 180°.

5. The movement mechanism according to any one of the preceding claims, the hollow body (5) having surface sections connected via the receiving area wall (11) which enclose an acute angle around the receiving area (2).

6. The movement mechanism according to any one of the preceding claims, sides of the fluid volume (7) facing the receiving area (2) and sides of the fluid volume (7) facing away from the receiving area (2) running parallel to each other.

7. The movement mechanism according to any one of the preceding claims, the hollow body (5) having an outer surface that includes a cylindrical region (13) with a cross-section in a plane perpendicular to a longitudinal direction of the receiving area (2) in the shape of an arc with a straight section connecting ends of the arc or that has a rectangular shape,
the outer surface in a region with a straight cross-section having a cutout (15) parallel to the longitudinal direction of the receiving area (2), in which the outer surface (13) merges into the receiving area wall (11).

8. The movement mechanism according to any one of the preceding claims, comprising a foil (4) which completely surrounds the hollow body (5), preferably below the receiving area (2), and which extends in a plane parallel to the longitudinal direction of the receiving area (2).

9. The movement mechanism according to any one of the preceding claims, the receiving area wall (11) having the shape of a two-dimensional U in a section in a plane perpendicular to an extension direction of the receiving area (2).

10. The movement mechanism according to any one of the preceding claims, further comprising at least one centering element (91, 92) arranged on at least one side of the receiving area (2) towards its longitudinal direction, the elongated object (3, 17) can be centred on a central axis of the receiving area (2), the at least one centering element (91, 92) preferably having a channel (94) or a through-hole (93) that runs coaxially to the central axis of the receiving area (2).

11. The movement mechanism according to any one of the preceding claims, comprising at least two of the clamping actuators (1a, 1b), whose receiving areas (2a, 2b) are coaxial with each other,
further comprising at least one of the movement actuators (9a, 9b, 9d, 9e) for each of the clamping actuators (1a, 1b).

12. The movement mechanism according to any one of the preceding claims, the movement mechanism having a U-shaped, channel-shaped, or concave receptacle,
the clamping actuator being arranged between legs of the receptacle,
an axis around which the receptacle extends being parallel to an axis around which the receiving area wall (11) extends.

## Revendications

1. Mécanisme de mouvement pour mettre en mouvement au moins un objet allongé (3, 17),
dans lequel le mécanisme de mouvement présente au moins un actionneur de serrage (1) pour retenir un objet allongé (3,17),
dans lequel l'actionneur de serrage (1)
présente un corps creux (5) avec un volume de fluide (7),
dans lequel le corps creux (5) présente une paroi de zone de réception (11) en forme de U, en forme de gouttière ou concave, qui fait le tour au moins partiellement d'une zone de réception (2) allongée dans laquelle l'objet allongé (3, 17) peut être inséré,
dans lequel la paroi de zone de réception (11) sépare la zone de réception (2) du volume de fluide (7),
dans lequel la paroi de zone de réception (11) est formée par une membrane flexible,
dans lequel l'actionneur de serrage (1) présente en outre un raccord de fluide (6), par lequel du fluide peut être introduit dans le volume de fluide (7) et peut être évacué du volume de fluide (7), et
dans lequel le mécanisme de mouvement présente en outre au moins un actionneur de mouvement (9ra, 9rb, 9t) avec lequel l'actionneur de serrage (1) peut se déplacer en direction d'une direction longitudinale de la zone de réception (2) et/ou peut tourner autour de la zone de réception (2),
dans lequel le mécanisme de mouvement selon la variante A présente au moins un premier et un deuxième des actionneurs de serrage (1a, 1b),
dans lequel le premier actionneur de serrage (1a) est disposé sur un premier actionneur de mouvement (9a, 9b) et le deuxième actionneur de serrage (1b) est disposé sur un deuxième actionneur de mouvement (9d, 9e),
dans lequel les actionneurs de serrage (1a, 1b) peuvent se déplacer avec les actionneurs de mouvement (9a, 9b, 9d, 9e) en direction de la direction longitudinale de leurs zones de réception (2a, 2b),
dans lequel le mécanisme de mouvement est configuré pour mettre en œuvre le procédé suivant : dans lequel les actionneurs de serrage (1a, 1b) et les actionneurs de mouvement (9a, 9d, 9b, 9e) sont actionnés selon un modèle d'actionnement après l'insertion de l'objet allongé (3,17) dans les zones de réception (2a, 2b) des deux actionneurs de serrage (1a, 1b),
dans lequel le modèle d'actionnement comprend, de manière répétée, les étapes suivantes, qui peuvent également se chevaucher :
1) le premier actionneur de serrage (1a) est actionné dans une première position initiale, de sorte qu'il saisit l'objet allongé (3,17),
2) le premier actionneur de mouvement (9a, 9b) met en mouvement le premier actionneur de serrage (1a) dans une direction de transport,
3) le premier actionneur de serrage (1a) termine son actionnement de sorte qu'il libère l'objet allongé (3, 17), et le deuxième actionneur de serrage (1b) est actionné dans une deuxième position initiale de sorte qu'il saisit l'objet (3, 17),
4) le deuxième actionneur de mouvement (9d, 9e) met en mouvement le deuxième actionneur de serrage (1b) dans la direction de transport et le premier actionneur de mouvement (9a, 9b) ramène le premier actionneur de serrage (1a) dans la première position initiale, dans le sens inverse de la direction de transport,
5) le deuxième actionneur de serrage (1b) termine son actionnement de sorte qu'il libère l'objet allongé (3, 17) et est mis en mouvement vers la deuxième position initiale par le deuxième actionneur de mouvement (9d, 9e),
et/ou
dans lequel, pour le mécanisme de mouvement selon la variante B, le premier actionneur de serrage (1a) est disposé sur un premier actionneur de rotation et le deuxième actionneur de serrage (1b) est disposé sur un deuxième actionneur de rotation, dans lequel les actionneurs de serrage (1a, 1b) peuvent tourner avec les actionneurs de rotation respectifs autour de l'axe longitudinal de leurs zones de réception (2a, 2b), dans lequel le mécanisme de mouvement est configuré pour mettre en œuvre le procédé suivant :
dans lequel les actionneurs de serrage (1a, 1b) sont actionnés selon un modèle d'actionnement après insertion de l'objet allongé (3,17) dans les zones de réception (2a, 2b) des deux actionneurs de serrage (1a, 1b) et des actionneurs de rotation, dans lequel le modèle d'actionnement comprend, de manière répétée, les étapes suivantes, qui peuvent également se chevaucher :
1) le premier actionneur de serrage (1a) est actionné dans une première position angulaire initiale, de sorte qu'il saisit l'objet allongé (3,17),
2) le premier actionneur de rotation fait alors tourner le premier actionneur de serrage (1a) autour de l'axe longitudinal de la zone de réception (2a) ou de l'objet allongé (3,17) dans un sens de rotation souhaité,
3) le premier actionneur de serrage (1a) termine alors son actionnement de sorte qu'il libère l'objet allongé (3, 17), et le deuxième actionneur de serrage (1b) est actionné dans une deuxième position angulaire initiale,
4) le deuxième actionneur de rotation fait alors tourner le deuxième actionneur de serrage (1b) dans le sens de rotation souhaité, et le premier actionneur de rotation ramène en outre par rotation le premier actionneur de serrage (1a) dans la position angulaire initiale à l'encontre du sens de rotation souhaité,
5) le deuxième actionneur de serrage (1b) termine alors son actionnement, de sorte qu'il libère l'objet allongé (3,17) et est, au moins dans la mesure où un autre passage a lieu, ramené dans la deuxième position angulaire initiale par le deuxième actionneur de rotation.

2. Mécanisme de mouvement selon la revendication précédente, dans lequel la membrane flexible délimite tous les côtés du volume de fluide (7), et/ou
dans lequel la zone de réception (2) s'étend le long d'une droite.

3. Mécanisme de mouvement selon l'une quelconque des revendications précédentes, dans lequel la membrane flexible, dans un état dans lequel aucune force extérieure n'agit sur elle, a une forme qui est obtenue par déplacement d'une came plate fermée le long d'une course de déplacement courbée autour d'un axe médian, dans lequel l'axe médian se situe, à chaque position du déplacement, à l'extérieur de la surface entourée par la came et dans le plan de la came,
dans lequel les extrémités de la course de déplacement forment un angle supérieur à zéro autour de l'axe médian,
dans lequel de préférence la zone de réception s'étend de manière allongée le long de l'axe médian,
dans lequel de préférence la course de déplacement fait le tour de l'axe médian de plus de 180 degrés, de préférence de plus de 270 degrés.

4. Mécanisme de mouvement selon l'une quelconque des revendications précédentes, dans lequel la paroi de zone de réception (11) fait le tour de la zone de réception (2) sur un angle supérieur à 180 °.

5. Mécanisme de mouvement selon l'une quelconque des revendications précédentes, dans lequel le corps creux (5) présente des parties de surface reliées par la paroi de zone de réception (11), qui forment un angle aigu autour de la zone de réception (2).

6. Mécanisme de mouvement selon l'une quelconque des revendications précédentes, dans lequel les côtés du volume de fluide (7) tournés vers la zone de réception (2) et les côtés du volume de fluide (7) opposés à la zone de réception (2) sont parallèles entre eux.

7. Mécanisme de mouvement selon l'une quelconque des revendications précédentes, dans lequel le corps creux (5) présente une surface extérieure qui présente une zone cylindrique (13) avec une section transversale dans un plan perpendiculaire à une direction longitudinale de la zone de réception (2), qui a la forme d'un arc de cercle avec une partie rectiligne reliant les extrémités de l'arc de cercle ou qui a une forme rectangulaire,
dans lequel la surface extérieure présente, dans une zone à section transversale rectiligne, une incision (15) parallèle à la direction longitudinale de la zone de réception (2), dans laquelle la surface extérieure (13) se fond dans la paroi de zone de réception (11).

8. Mécanisme de mouvement selon l'une quelconque des revendications précédentes, présentant un film (4) qui fait complètement le tour du corps creux (5), de préférence en dessous de la zone de réception (2), et qui s'étend dans un plan parallèle à la direction longitudinale de la zone de réception (2).

9. Mécanisme de mouvement selon l'une quelconque des revendications précédentes, dans lequel la paroi de zone de réception (11) a la forme d'un U bidimensionnel dans une coupe dans un plan perpendiculaire à une direction d'une extension de la zone de réception (2).

10. Mécanisme de mouvement selon l'une quelconque des revendications précédentes, présentant en outre au moins un élément de centrage (91, 92) qui est disposé sur au moins un côté de la zone de réception (2) en direction de sa direction longitudinale, dans lequel l'objet allongé (3,17) peut être centré sur un axe médian de la zone de réception (2) avec l'au moins un élément de centrage (91, 92), dans lequel l'au moins un élément de centrage (91, 92) présente de préférence une rainure (94) ou un trou débouchant (93) qui s'étend coaxialement à l'axe médian de la zone de réception (2).

11. Mécanisme de mouvement selon l'une quelconque des revendications précédentes, présentant au moins deux des actionneurs de serrage (1a, 1b) dont les zones de réception (2a, 2b) se situent coaxialement entre elles,
présentant en outre au moins un des actionneurs de mouvement (9a, 9b, 9d, 9e) pour chacun des actionneurs de serrage (1a, 1b).

12. Mécanisme de mouvement selon l'une quelconque des revendications précédentes, dans lequel le mécanisme de mouvement présente un logement en forme de U, en forme de gouttière ou concave,
dans lequel l'actionneur de serrage est disposé entre des branches du logement,
dans lequel un axe, autour duquel le logement s'étend, se situe parallèlement à un axe autour duquel la paroi de zone de réception (11) s'étend.
